# EUROPEAN PATENT SPECIFICATION

(11) **EP 3 096 369 B1**
(45) Date of publication and mention of the grant of the patent: **14.10.2020**
(21) Application number: 15737444.8
(22) Date of filing: 16.01.2015
(51) Int. Cl.: H01L 51/46

(54) **ORGANIC SOLAR CELL AND METHOD FOR MANUFACTURING SAME**
ORGANISCHE SOLARZELLE UND VERFAHREN ZUR HERSTELLUNG DAVON
CELLULE SOLAIRE ORGANIQUE ET SON PROCÉDÉ DE FABRICATION

(30) Priority: 17.01.2014 KR 20140005862
(43) Date of publication of application: 23.11.2016
(73) Proprietor: LG Chem, Ltd., Seoul 150-721 (KR)
(72) Inventor: CHO, Keun, Daejeon 305-738 (KR); CHOI, Jeong Min, Daejeon 305-738 (KR); BAE, Jaesoon, Daejeon 305-738 (KR); LEE, Jaechol, Daejeon 305-738 (KR); LEE, Jiyoung, Daejeon 305-738 (KR); KIM, Jinseck, Daejeon 305-738 (KR)
(74) Representative: Hoffmann Eitle
(86) International application number: PCT/KR2015/000500
(87) International publication number: WO 2015/108374

(56) References cited:
- JP-A- H0 948 929
- JP-A- 2008 226 959
- KR-A- 20090 113 574
- KR-A- 20120 018 808
- KR-A- 20120 059 573
- KR-A- 20130 118 218
- SHABI THANKARAJ SALAMMAL ET AL: "Influence of Solubilizing Group Removal Rate on the Morphology and Crystallinity of a Diketopyrrolopyrrole-Based Compound", CRYSTAL GROWTH & DESIGN., vol. 14, no. 1, 2 December 2013 (2013-12-02), pages 339-349, XP055390092, US ISSN: 1528-7483, DOI: 10.1021/cg401625p
- YUKI SUNA ET AL: "Ambipolar Behavior of Hydrogen-Bonded Diketopyrrolopyrrole-Thiophene Co-oligomers Formed from Their Soluble Precursors", ORGANIC LETTERS , 14(23), 6012-6015 CODEN: ORLEF7; ISSN: 1523-7052, vol. 14, no. 13, 20 June 2012 (2012-06-20) , pages 3356-3359, XP055339608, ISSN: 1523-7060, DOI: 10.1021/ol3013364
- JUNGHOON LEE ET AL: "Inversion of Dominant Polarity in Ambipolar Polydiketopyrrolopyrrole with Thermally Removable Groups", ADVANCED FUNCTIONAL MATERIALS, vol. 22, no. 19, 12 June 2012 (2012-06-12) , pages 4128-4138, XP055113862, ISSN: 1616-301X, DOI: 10.1002/adfm.201200940

## Description

### [Technical Field]

The present specification claims priority from Korean Patent Application No. 10-2014-0005862 filed on January 17, 2014 at the KIPO.

The present invention relates to an organic solar cell and a method for preparing the same.

### [Background Art]

A solar cell is a device that may convert the solar energy directly into electrical energy by applying a photovoltaic effect. The solar cell may be divided into an inorganic solar cell and an organic solar cell according to the material which constitutes a thin film. A typical solar cell is prepared with a p-n junction obtained by doping crystalline silicon (Si), which is an inorganic semiconductor. Electrons and holes generated due to absorption of light diffuse to a p-n junction point, and are accelerated by an electric field and moved to an electrode. A power conversion efficiency of this procedure is defined as a ratio of a power given in an external circuit to a solar power fed into the solar cell, and reaches up to 24% when measured under the virtual solar irradiation conditions currently standardized. However, since the inorganic solar cell in the related art already shows limitations in economic feasibility and available materials, an organic semiconductor solar cell, which is easily processed and cheap and has various functionalities, is in the spotlight as a long-term alternative energy source.

Since the organic solar cell uses various organic semiconductor materials in small amounts, material costs may be reduced and a thin film may be prepared by a wet process, so that it is possible to prepare a device by an easy method.

An organic solar cell is classified into a bi-layer p-n junction type organic solar cell including a photoactive layer composed of two layers of a p-type semiconductor thin film and an n-type semiconductor thin film and a bulk heterojunction (BHJ) type organic solar cell including a photoactive layer in which an n-type semiconductor and a p-type semiconductor are blended, depending on the structure of the photoactive layer.

In the solar cell, it is important to increase the efficiency such that electrical energy as much as possible may be outputted from the solar energy. In order to increase the efficiency of the solar cell, it is also important to generate excitons as much as possible from the inside of the semiconductor, but it is also important to take out electric charges generated to the outside without being lost. One of the causes that electric charges are lost is that generated electrons and holes are annihilated by means of recombination. As a method of transferring generated electrons or holes to an electrode without being lost, various methods have been suggested, but most of the methods require additional processes, and as a result, preparation costs may be increased.

### [Citation List]

### [Patent Document]

Official Gazette of Korean Patent Application Laid-Open No. 10-2010-0011186

JP 2008 226959

SHABI T. SALAMMAL ET AL:"Influence of Solubilizing Group Removal Rate on the Morphology and Crystallinity of a Diketopyrrolopyrrole-Based Compound", CRYSTAL GROWTH & DESIGN., vol. 14, no. 1, 2 December 2013, pages 339-349

YUKI SUNA ET AL: "Ambipolar Behavior of Hydrogen-Bonded Diketopyrrolopyrrole-Thiophene Co-oligomers Formed from Their Soluble Precursors",ORGANIC LETTERS , 14(23), 6012-6015, vol. 14, no. 13, 20 June 2012, pages 3356-3359

JUNGHOON LEE ET AL: "Inversion of Dominant Polarity in Ambipolar Polydiketopyrrolopyrrole with Thermally Removable Groups",ADVANCED FUNCTIONAL MATERIALS, vol. 22, no. 19, 12 June 2012, pages 4128-4138

### [Detailed Description of the Invention]

### [Technical Problem]

An object of the present invention is to provide a material for a photoactive layer which may have high hole mobility to exhibit excellent electrical characteristics, and is excellent particularly in photoelectric conversion efficiency characteristics by having a stable HOMO energy level to have a high open voltage, and an organic solar cell including the same. Further, a high photoelectric conversion efficiency may be obtained by increasing mobility of holes through heat treatment. In addition, another object of the present invention is to provide an electron donor material which may be mass-produced because the preparation process is simple.

### [Technical Solution]

An exemplary embodiment of the present invention provides an organic solar cell : according to claim 1.

Further, another exemplary embodiment of the present invention provides a method for preparing an organic solar cell, the method being according to claim 7.

### [Advantageous Effects of Invention]

An organic solar cell according to the present invention may be prepared by a solution process, and may include a photoactive layer which has a strong binding force due to a hydrogen bond and is excellent in crystallinity. Accordingly, the organic solar cell according to the present invention may exhibit characteristics which are excellent in driving voltage, current, service life, and the like.

### [Description of Drawings]

FIG. 1 a view illustrating UV spectrum results of Formula 1-1 after a heat treatment process according to an exemplary embodiment of the present invention.
FIG. 2 is a view illustrating an NMR result of Compound 1 of Preparation Example 1 according to an exemplary embodiment of the present invention.
FIG. 3 is a view illustrating MS data of Compound 1 of Preparation Example 1 according to an exemplary embodiment of the present invention.
FIG. 4 is a view illustrating an NMR result of Compound 2 of Preparation Example 2 according to an exemplary embodiment of the present invention.
FIG. 5 is a view illustrating MS data of Compound 2 of Preparation Example 1 according to an exemplary embodiment of the present invention.
FIGS. 6 and 7 are views illustrating NMR results of Compound 3 of Preparation Example 1 according to an exemplary embodiment of the present invention.
FIG. 8 is a view illustrating MS data of Formula 1-2 of Preparation Example 2 according to an exemplary embodiment of the present invention.
FIG. 9 is a view illustrating an UV spectrum of Formula 1-1 according to an exemplary embodiment of the present invention.
FIG. 10 is a view illustrating a thermogravimetry analysis of Formula 1-1 according to an exemplary embodiment of the present invention.
FIG. 11 is a view illustrating an IR spectrum of Formula 1-1 according to an exemplary embodiment of the present invention.
FIG. 12 is a view illustrating a current density according to the voltage of an organic solar cell according to Comparative Example 1.
FIG. 13 is a view illustrating a current density according to the voltage of an organic solar cell according to Experimental Example 1.

### [Best Mode]

Hereinafter, the present invention will be described in detail.

When one member is positioned "on" another member, the present specification includes not only the case where the one member is adjacent to the another member, but also the case where a third member is present between the two members.

An organic solar cell according to an exemplary embodiment of the present invention includes an anode, a cathode, and an organic material layer having one or more layers disposed between the anode and the cathode,
in which at least one of the organic material layer having one or more layers includes a : compound represented by the Formula 2-1 or Formula 2-2.

The organic solar cell according to an exemplary embodiment of the present specification includes an anode, a cathode, and an organic material layer having one or more layers disposed between the anode and the cathode, and the organic material layer having one or more layers includes an organic material layer formed by a method including forming a thin film by using a compound into which a substituent (a leaving group) to be removed by heat treatment, light treatment, or acid treatment is introduced, and then performing heat treatment, light treatment, or acid treatment, and
the organic material layer formed by performing heat treatment, light treatment, or acid treatment includes a compound having a group capable of forming a hydrogen bond.

In an exemplary embodiment of the present specification, the compound from which a leaving group has been removed after heat treatment, light treatment, or acid treatment is present in an amount of 90% or more based on the total solid content of the organic material layer formed by performing heat treatment, light treatment, or acid-treatment.

In the present specification, the group capable of forming a hydrogen bond means a functional group which may form a hydrogen bond.

Further, in the present specification, the leaving group means a substituent to be removed by heat treatment, light treatment, or acid treatment.

The organic solar cell according to an exemplary embodiment of the present specification includes an anode, a cathode, and an organic material layer having one or more layers disposed between the anode and the cathode,
in which at least one of the organic material layer having one or more layers is formed by using a compound having a group capable of forming a hydrogen bond, and a leaving group, and the leaving group is removed by heat treatment, light treatment, or acid treatment.

In the organic material layer according to an exemplary embodiment of the present specification, a hydrogen atom instead of the leaving group is substituted while the leaving group is removed, and the substituted hydrogen atom may form a hydrogen bond with a group capable of forming a hydrogen bond in and/or out of the molecule.

In general, a material for an organic material layer, which is used in the preparation of an organic solar cell, forms a packing only by interaction due to the van der Waals energy, and thus is disadvantageous in that the intermolecular attraction is not sufficiently strong, and as a result, the intermolecular binding force is weak and the intermolecular distance is not sufficiently close, and accordingly, movement of the intermolecular carrier is not sufficiently high. Further, since the intermolecular packing is not sufficient, air or moisture is easily introduced into the organic material layer from the outside, thereby showing a tendency that stability of a device deteriorates.

In order to solve the disadvantage, the present specification forms an organic material layer by using a compound which may form a stronger hydrogen bond than a van der Waals bond having a weak binding force between materials in an organic material layer in preparing an organic solar cell.

In general, a hydrogen bond is produced by interaction of hydrogen bound to an atom having large electronegativity, such as O, N, S, and F, with an unshared electron pair of an atom, such as O, N, S, and F of another adjacent molecule. Since the electronegativity of O, N, S, F, and the like is significantly larger than the electronegativity of a hydrogen atom, a positive charge is partially produced in a hydrogen atom, and a partial negative charge is produced in an atom such as O, N, S, and F. Since the partially positively charged hydrogen atom exhibits strong interaction with an adjacent atom such as oxygen, nitrogen, sulfur, and fluorine, which is negatively charged, the distance between the two molecules is very closely packed, and is easily uniformly arranged. For the intermolecular packing, movement of the carrier in the photoactive layer may be facilitated by reducing the intermolecular distance. Further, the photoactive layer by a hydrogen bond may increase stability of the organic solar cell by the air or moisture.

In the organic solar cell according to the present specification, the organic material layer may be formed by a method known in the art using a compound having a group capable of forming a hydrogen bond.

In an exemplary embodiment of the present specification, in the organic material layer, the compound before being subjected to heat treatment, light treatment, or acid treatment includes a group capable of forming a hydrogen bond, and is formed by using a compound into which a leaving group is introduced.

Typically, a method of forming a photoactive layer during the preparation of an organic solar cell may be divided into a vacuum deposition method and a solution application method. Specifically, since the vacuum deposition method requires a high temperature and a high degree of vacuum, it is difficult to achieve a large area, the equipment is relatively expensive, and the process is relatively complex. In contrast, the solution application method typified by a spin coating method, an inkjet printing method, a dip coating method, a roll coating method, a screen printing method, and the like may form a thin film having a large area, and makes a process under normal temperature and normal pressure possible, and thus, is advantageous in that a process cost may be reduced.

However, compounds having a strong intermolecular interaction make infiltration of an organic solvent in the molecule difficult, and thus have a tendency that the solubility of the material is low.

Therefore, in order to use the advantage of a hydrogen bond between the materials of an organic material layer in preparing an organic solar cell, the present specification provides a method for forming an organic material layer by forming the organic material layer with a compound including a group capable of forming a hydrogen bond to increase the intermolecular packing and the crystallinity and using a vacuum deposition method, or forming the organic material layer by a solution process by introducing a bulky substitution product into a group capable of forming a hydrogen bond to increase the solubility of a material for the organic material layer such that an organic solvent is easily infiltrated into the molecule.

Specifically, the organic material layer of the organic solar cell according to the present specification may be formed by a compound having a group capable of forming a hydrogen bond.

That is, the intermolecular interaction is weakened by introducing a substituent, which may be removed by heat, light, or acid, into the functional group of a compound bound to N, O, S, and F atoms, and the like and including a group capable of forming a hydrogen bond to remove a hydrogen bond. In particular, when the substituent is a substituent which may increase the solubility to a solvent, for example, a bulky substituent, the solubility of the compound is increased, and the compound may be easily prepared as a solution, so that a thin film may be formed by a solution application method using the prepared solution. Subsequently, an organic semiconductor material having a strong binding force, which is capable of forming a hydrogen bond again, is produced by subjecting the formed thin film to heat, light, or acid treatment to remove the substituent. Therefore, while the above-described method for preparing an organic solar cell according to the present specification forms a thin film by a solution process, the resultantly formed film has high intermolecular packing and crystallinity by a hydrogen bond, and simultaneously has excellent stability to moisture or the air.

In the present specification, as the compound having a group capable of forming a hydrogen bond, a compound, which is capable of forming a hydrogen bond and may serve as a semiconductor by the above-described action principle, may be used without limitation in kind thereof.

In the present specification, the group capable of forming a hydrogen bond is selected from the group consisting of: an alcohol group; a phenol group; a catechol group; a carbonyl group; a thiol group; and an amine group.

In an exemplary embodiment of the present specification, the compound before being subjected to heat treatment, light treatment, or acid treatment is a compound which includes a group capable of forming a hydrogen bond and into which a leaving group is introduced.

In the present specification, the compound into which a leaving group is introduced means that hydrogen is substituted with a leaving group, and means a compound from which the leaving group is removed and which is substituted with hydrogen, when the compound is treated with heat, light, or acid, a compound.

In an exemplary embodiment of the present specification, an alcohol group, a phenol group, a catechol group, a carbonyl group, a thiol group, an amine group, and the like, which are the group capable of forming a hydrogen bond, may form an ether bond, an ester bond, a thioether bond, a thioester bond, and an amide bond with the leaving group to introduce a leaving group.

In the present specification, a leaving group introduced into each group capable of forming a hydrogen bond will be described below, but is not limited thereto.

When the group capable of forming a hydrogen bond is an alcohol group, a leaving group introduced into the alcohol group includes methyl ether, methoxymethyl ether, methoxythiomethyl ether, 2-methoxyethoxymethyl ether, bis(2-chloroethoxy)methyl ether, tetrahydropyranyl ether, 4-methoxytetrahydropyranyl ether, tetrahydrothiopyranyl ether, 4-methoxytetrahydropyranyl ether, 4-methoxythetrahydrothiopyranyl ether, tetrahydrofuranyl ether, tetrahydrothiofuranyl ether, 1-ethoxyethyl ether, 1-methyl-1-methoxyethyl ether, 2-(phenylselenyl)ethyl ether, t-butyl ether, allyl ether, benzyl ether, o-nitrobenzyl ether, triphenylmethyl ether, anaphthyldiphenylmethyl ether, p-methoxyphenyldiphenylmethyl ether, 9-(9-phenyl-10-oxo)anthryl ether, trimethylsilyl ether, isopropyldimethylsilyl ether, t-butyldimethylsilyl ether, t-butyldiphenylsilyl ether, tribenzylsilyl ether, triisopropylsilyl ether, formate ester, acetate ester, trichloroacetate ester, phenoxyacetate ester, isobutyrate ester, pivaloate ester, adamantoate ester, benzoate ester, 2,4,6-trimethylbenzoate ester, methyl carbonate, 2,2,2-trichloroethyl carbonate, allyl carbonate, p-nitrophenyl carbonate, benzyl carbonate, s-benzyl thiocarbonate, N-phenyl carbonate, nitrate ester, 2,4-dinitrophenylsulfenate ester, and the like.

Preferably, when the group capable of forming a hydrogen bond is an alcohol group, a leaving group introduced into the alcohol group includes trichloroacetate ester, methyl carbonate, 2,2,2-trichloroethyl carbonate, allyl carbonate, p-nitrophenyl carbonate, benzyl carbonate, s-benzyl thiocarbonate, nitrate ester, and 2,4-dinitrophenylsulfenate ester.

More preferably, when the group capable of forming a hydrogen bond is an alcohol group, a leaving group introduced into the alcohol group includes nitrate ester and 2,4-dinitrophenylsulfenate ester.

When the group capable of forming a hydrogen bond is a phenol group or a catechol group, a leaving group introduced into the phenol group or the catechol group includes methyl ether, methoxymethyl ether, 2-methoxyethoxymethyl ether, methoxythiomethyl ether, penacyl ether, allyl ether, cyclohexyl ether, t-butyl ether, benzyl ether, o-nitrobenzyl ether, 9-anthrylmethyl ether, 4-picolyl ether, t-butyldimethylsilyl ether, aryl acetate, aryl pivaloate, aryl benzoate, aryl 9-fluorenecarboxylate, aryl methyl carbonate, aryl 2,2,2-trichloroethyl carbonate, aryl vinyl carbonate, aryl benzyl carbonate, aryl methanesulfonate, methylenedioxy derivatives, acetonide derivatives, diphenylmethylenedioxy derivatives, cyclic borate, cyclic carbonate, and the like.

When the group capable of forming a hydrogen bond is a carbonyl group, a leaving group introduced into the carbonyl group includes dimethyl acetal and ketal, bis(2,2,2-trichloroethyl) acetal and ketal, 1,3-dioxanes, 5-methylene-1,3-dioxanes, 5,5-dibromo-1,3-dioxanes, 1,3-dioxolanes, 4-bromomethyl-1,3-dioxolanes, 4-o-nitrophenyl-1,3-dioxolanes, S,S'-dimethyl acetal and ketal, 1,3-dithianes, 1,3-dithiolane, 1,3-oxathiolanes, o-trimethylsilyl cyanohydrins, N,N-dimethylhydrazones, 2,4-dinitrophenylhydrazones, o-phenylthiomethyl oximes, substituted methylene derivatives, bismethylenedioxy derivatives, methyl ester, methoxymethyl ester, methylthiomethyl ester, tetrahydropiranyl ester, benzyloxymethyl ester, penacyl ester, N-phthalimidomethyl ester, 2,2,2-trichloroethyl ester, 2-haloethyl ester, 2-(p-toluenesulfonyl)ethyl ester, t-butyl ester, cinnamyl ester, benzyl ester, triphenylmethyl ester, bis(o-nitrophenyl)methyl ester, 9-anthrylmethyl ester, 2-(9,10-dioxo)anthrylmethyl ester, piperonyl ester, trimethylsilyl ester, t-butyldimethylsilyl ester, s-t-butyl ester, 2-alkyl-1,3-oxazolines, N,N-dimethylamide, N-7-nitroindoylamide, hydrazide, N-phenylhydrazide, N,N'-diisopropylhydrizide, and the like.

When the group capable of forming a hydrogen bond is a thiol group, a leaving group introduced into the thiol group includes s-benzyl thioether, s-p-methoxybenzyl thioether, s-p-nitrobenzyl thioether, s-4-picolyl thioether, s-2-picolyl N-oxide thioether, s-9-anthrylmethyl thioether, s-diphenylmethyl thioether, s-di(p-methoxyphenyl)methyl thioether, s-triphenylmethyl thioether, s-2,4-dinitrophenyl thioether, s-t-butyl thioether, s-isobutoxymethyl monothioacetal, s-2-tetrahydropiranyl monothioacetal, s-acetamidomethyl aminothioacetal, s-cyanomethyl thioether, s-2-nitro-1-phenylethyl thioether, s-2,2-bis(carboethoxy)ethyl thioether, s-benzoyl derivatives, s-(N-ethylcarbamate), s-ethyl disulfide, and the like.
When the group capable of forming a hydrogen bond is an amine group, a leaving group introduced into the amine group includes methyl carbamate, 9-fluorenylmethyl carbamate, 2,2,2-trichloroethyl carbamate, 2-trimethylsilyl carbamate, 1,1-dimethylpropynyl carbamate, 1-methyl-1-phenylethyl carbamate, 1-methyl-1-(4-biphenylyl)ethyl carbamate, 1,1-dimethyl-2-haloethyl carbamate, 1,1-dimethyl-2-cyanoethyl carbamate, t-butyl carbamate, cyclobutyl carbamate, 1-methylcyclobutyl carbamate, 1-adamantyl carbamate, vinyl carbamate, allyl carbamate, cinnamyl carbamate, 8-quinolyl carbamate, N-hydroxypiperidinyl carbamate, 4,5-diphenyl-3-oxazoline-2-one, benzyl carbamate, p-nitrobenzyl carbamate, 3,4-dimethoxy-6-nitrobenzyl carbamate, 2,4-dichlorobenzyl carbamate, 5-benzisoxazolylmethyl carbamate, 9-anthrylmethyl carbamate, diphenylmethyl carbamate, isonicotinyl carbamate, s-benzyl carbamate, N-(N'-phenylaminothiocarbonyl) derivatives, N-formyl, N-acetyl, N-chloroacetyl, N-trichloroacetyl, N-trifluoroacetyl, N-o-nitrophenylacetyl, N-o-nitrophenoxyacetyl, N-acetoacetyl, N-3-phenylpropionyl, N-3-(p-hydroxyphenyl)propionyl, N-2-methyl-2-(o-nitrophenoxy)propionyl, N-2-methyl-2-(o-phenylazophenoxy)propionyl, N-4-chlorobutyryl, N-o-nitrocinnamoyl, N-picolinoyl, N-(N'-acetylmethionyl), N-benzoyl, N-phthaloyl, N-dithiasuccinoyl, N-allyl, N-phenacyl, N-3-acetoxypropyl, quaternary ammonium salts, N-methoxymethyl, N-benzyloxymethyl, N-pivaloyloxymethyl, N-tetrahydropyranyl, N-2,4-dinitrophenyl, N-benzyl, N-o-nitrobenzyl, N-di(p-methoxyphenyl) methyl, N-triphenylmethyl, N-(p-methoxyphenyl)diphenylmethyl, N-diphenyl-4-pyridylmethyl, N-2-picolyl N'-oxide, N,N'-isopropylidene, N-benzylidene, N-p-nitrobenzylidene, N-salicylidene, N-(5,5-dimethyl-3-oxo-1-cyclohexenyl, N-nitro, N-oxide, N-diphenylphosphinyl, N-dimethylthiophosphinyl, N-benzenesulfenyl, N-o-nitrobenzenesulfenyl, N-2,4,6-trimethylbenzenesulfonyl, N-toluenesulfonyl, N-benzylsulfonyl, N-trifluoromethylsulfonyl, N-phenacyl sulfonyl, and the like.

Preferably, when the group capable of forming a hydrogen bond is an amine group, a leaving group introduced into the amine group includes 1,1-dimethyl-2-haloethyl carbamate, 1,1-dimethyl-2-cyanoethyl carbamate, t-butyl carbamate, and N-(N'-acetylmethionyl).

More preferably, when the group capable of forming a hydrogen bond is an amine group, a leaving group introduced into the amine group includes 1,1-dimethyl-2-haloethyl carbamate and t-butyl carbamate.

Most preferably, when the group capable of forming a hydrogen bond is an amine group, a leaving group introduced into the amine group is t-butyl carbamate.

In an exemplary embodiment of the present specification, the leaving group is a substituted or unsubstituted ester group.

In an exemplary embodiment of the present specification, the leaving group is a tert-butyl ester group (or t-butoxycarbonyl).

Hereinafter, the following examples will be described in order to describe a compound, which may be used in the present specification, in detail, but are provided only for better understanding and are not limited to these examples.

In the compound having a group capable of forming a hydrogen bond, the group capable of forming a hydrogen bond is a functional group represented by the following Formula 1. Specifically, in an exemplary embodiment of the present specification, the compound before being subjected to heat treatment, light treatment, or acid treatment is represented by the following Formula 1.

A compound represented by the following Formula 1 includes a ketone group and an NH group as the group capable of forming a hydrogen bond. The compounds represented by the following Formula 1 form hydrogen bonds with each other, and thus may produce a strong interaction. Therefore, these compounds having strong binding force, or a material for an organic material layer, which includes these compounds, may be formed as an organic material layer by vacuum deposition.

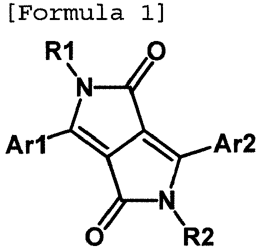

In Formula 1,
R1 and R2 are the same as or different from each other, and each independently selected from the group consisting of a substituted or unsubstituted ester group, a substituted or unsubstituted carbonyl group, a substituted or unsubstituted C₁ to C₂₀ alkyl group, a substituted or unsubstituted C₂ to C₂₀ alkenyl group, a substituted or unsubstituted C₃ to C₂₀ cycloalkyl group, a substituted or unsubstituted C₅ to C₃₀ cycloalkenyl group, a substituted or unsubstituted C₁ to C₃₀ alkoxy group, a substituted or unsubstituted C₆ to C₆₀ aryloxy group, a substituted or unsubstituted C₁ to C₃₀ alkylthioxy group, a substituted or unsubstituted C₅ to C₆₀ arylthioxy group, a substituted or unsubstituted C₁ to C₃₀ alkylamine group, a substituted or unsubstituted C₅ to C₆₀ arylamine group, a substituted or unsubstituted C₆ to C₆₀ aryl group, and a substituted or unsubstituted C₃ to C₆₀ heteroaryl group, and
Ar1 and Ar2 are substituted thiophene groups.

In an exemplary embodiment of the present specification, Formula 1 is represented by the following Formula 1A.

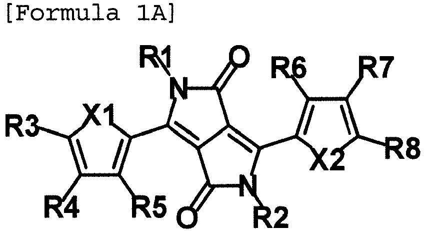

In Formula 1A,
R1 and R2 are the same as those defined in Formula 1,
R3 to R8 are the same as or different from each other, and each independently selected from the group consisting of hydrogen, substituted thiophene and unsubstituted benzothiophene and X1 and X2 are

When R3 and R8 are selected from substituted thiophene, than the substituents are selected from 2,9-dinaphthylantracenyl.

In an exemplary embodiment of the present specification, the compound before being subjected to heat treatment, light treatment, or acid treatment is represented by the following Formula 3.

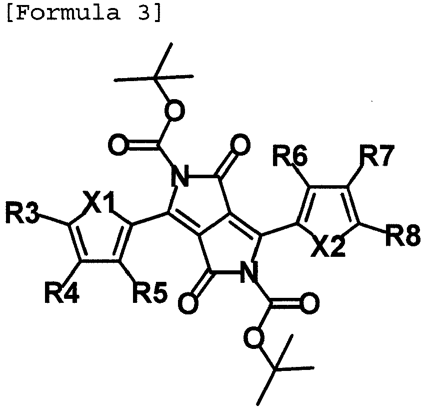

In Formula 3,
R3 to R8 are as defined above and
X1 and X2 are S. When a hydrogen atom which forms a hydrogen bond is substituted with a bulky substituent such as a tert-butyl ester group (or a t-butoxycarbonyl group) in the compound including a group capable of forming a hydrogen bond, a hydrogen bond fails to be formed as a compound of the following Formula 3.

Therefore, since the compound represented by Formula 3 fails to form a hydrogen bond due to introduction of a very bulky substituent, the intermolecular binding force becomes weak and the solubility is greatly increased so that the compound may be prepared as a solution, thereby enabling a thin film to be formed by a solution process. In an exemplary embodiment of the present specification, the compound before being subjected to heat treatment, light treatment, or acid treatment is represented by the following Formula 1-1 or 1-2.

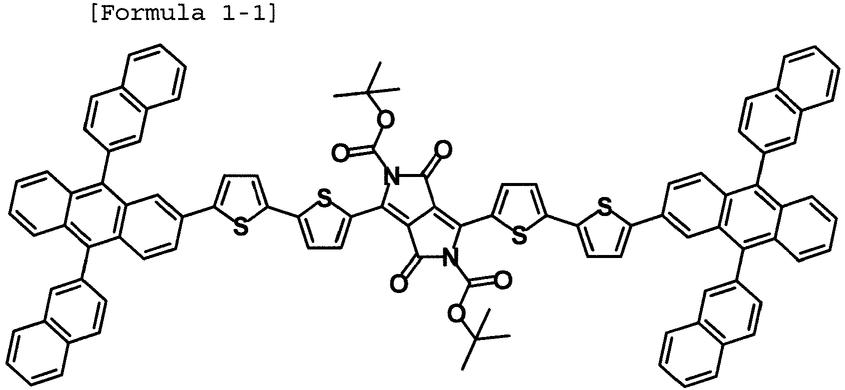

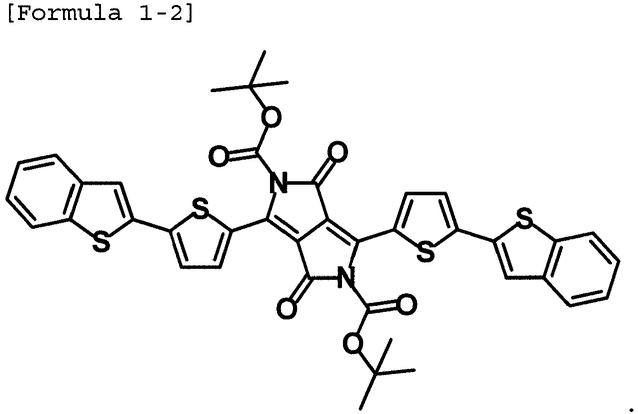

In the compound represented by Formula 3, a hydrogen atom is substituted with t-butoxycarbonyl, and a molecular bond is broken by heat treatment, light treatment, or acid treatment and the hydrogen bond may be substituted with a hydrogen bond.

The reformation of a hydrogen bond molecule by the heat treatment, light treatment, or acid treatment is a method commonly used in the protection and deprotection of a function group in an organic chemical reaction. In this case, a substituent to be substituted instead of the group capable of forming a hydrogen bond is not limited only to t-butoxycarbonyl which is a substituent of the compound represented by Formula 1-1, 1-2, or 3. In general, an alcohol group, a phenol group, a catechol group, a carbonyl group, a thiol group, an amine group, and the like, which are the group capable of forming a hydrogen bond, may by protected by an ether bond, an ester bond, a thioether bond, a thioester bond, an amide bond, and the like.

In an exemplary embodiment of the present specification, in the heat-treated, light-treated, or acid-treated compound, R1 and R2 in Formula 1 is hydrogen.

In an exemplary embodiment of the present specification, the heat-treated, light-treated, or acid-treated compound is represented by the following Formula 2.

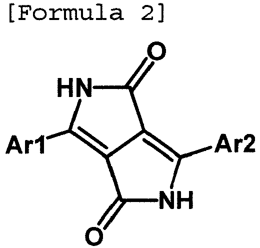

In Formula 2, Ar1 and Ar2 are the same as the definition described above.

In an exemplary embodiment of the present specification, the heat-treated, light-treated, or acid-treated compound is represented by the following Formula 4.

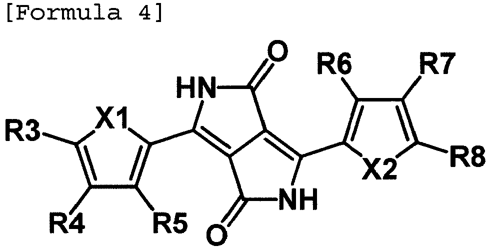

In Formula 4,
R3 to R8 are the same as defined above and
X1 and X2 are

Specifically, t-butoxycarbonyl is removed from the compound represented by Formula 3 by heat treatment, acid treatment, or light treatment, and a compound of Formula 4 is produced.

Therefore, since the compound represented by Formula 3 fails to form a hydrogen bond due to introduction of a very bulky substituent, the intermolecular binding force becomes weak and the solubility is increased so that the compound may be prepared, thereby enabling a thin film to be formed by a solution process.

In addition, after a thin film is formed, a compound represented by Formula 2 is formed by heat treatment, light treatment, or acid treatment, an intermolecular hydrogen bond having a strong binding force is formed, and the distance between the molecules is very closely packed and easily uniformly arranged.

Specifically, according to an exemplary embodiment of the present specification, the compound represented by Formula 1 is converted into the compound by Formula 2 by heat treatment, light treatment or acid treatment, and the compound represented by Formula 2 may form a hydrogen bond as described below.

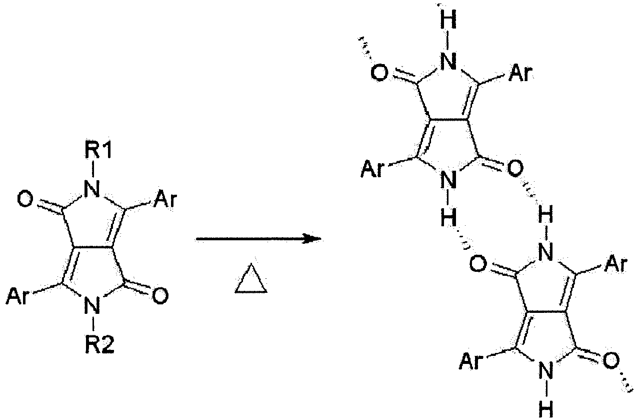

In the present invention, the heat-treated, light-treated, or acid-treated compound is represented by the following Formula 2-1 or 2-2.

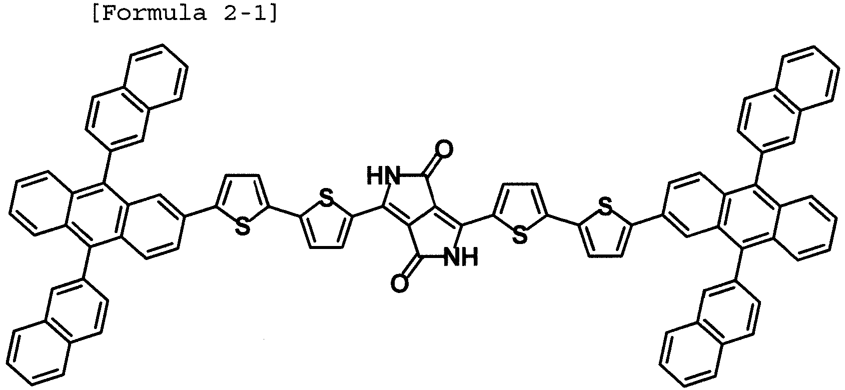

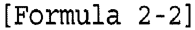

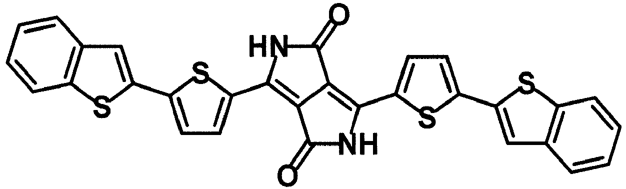

In the compound according to the present specification, the substituents will be described in more detail as follows.

As used herein, the term "substitution" (unless particularly specified, as it is the case of R3 and R8, for example) means that a hydrogen atom bound to a carbon atom of a compound is changed into another substituent, and the position to be substituted is not limited as long as the position is a position where the hydrogen atom is substituted, that is, a position where the substituent may be substituted, and when two or more hydrogen atoms are substituted, the two or more substituents may be the same as or different from each other.

Further, in the present specification, the term "substituted or unsubstituted" means (unless particularly specified, as it is the case of R3 and R8, for example) that a group is unsubstituted or substituted with one or more substituents selected from the group consisting of deuterium, a halogen group, an alkyl group, an alkenyl group, an alkoxy group, a silyl group, an arylalkenyl group, an aryl group, a heteroaryl group, a carbazole group, an arylamine group, an aryl group, and a nitrile group, or is unsubstituted or substituted with a substituent to which two or more substituents in the exemplified substituents are linked. For example, "a substituent to which two or more substituents are linked" may be a biphenyl group. That is, the biphenyl group may also be an aryl group, and may be interpreted as a substituent to which two phenyl groups are linked.

The alkyl group may be straight or branched, and the number of carbon atoms thereof is not particularly limited, but is preferably 1 to 20. Examples of the alkyl group or the alkylene group include a methyl group, an ethyl group, a propyl group, an isopropyl group, an n-butyl group, a s-butyl group, an isobutyl group, a t-butyl group, an n-pentyl group, an n-hexyl group, an n-heptyl group, an n-octyl group, a hydroxymethyl group, a 1-hydroxyethyl group, a 2-hydroxyethyl group, a 2-hydroxyisobutyl group, a 1,2-dihydroxyethyl group, a 1,3-dihydroxyisopropyl group, a 2,3-dihydroxy-t-butyl group, a 1,2,3 -trihydroxypropyl group, a chloromethyl group, a 1-chloromethyl group, a 2-chloromethyl group, a 2-chloroisobutyl group, a 1,2-dichloroethyl group, a 1,3-dichloroisopropyl group, a 2,3-dichloro-t-butyl group, a 1,2,3-trichloropropyl group, a bromomethyl group, a 1-bromoethyl group, a 2-bromoethyl group, a 2-bromoisobutyl group, a 1,2-dibromoethyl group, a 1,3-dibromoisopropyl group, a 2,3-dibromo-t-butyl group, a 1,2,3-tribromopropyl group, an iodomethyl group, a 1-iodoethyl group, a 2-iodoethyl group, a 2-iodoisobutyl group, a 1,2-diiodoethyl group, a 1,3-diiodoisopropyl group, a 2,3-diiodo-t-butyl group, a 1,2,3-triiodopropyl group, an aminomethyl group, a 1-aminoethyl group, a 2-aminoethyl group, a 2-aminoisobutyl group, a 1,2-diaminoethyl group, a 1,3-diaminoisopropyl group, a 2,3-diamino-t-butyl group, a 1,2,3-triaminopropyl group, a cyanomethyl group, a 1-cyanoethyl group, a 2-cyanoethyl group, a 2-cyanoisobutyl group, a 1,2-dicyanoethyl group, a 1,3-dicyanoisopropyl group, a 2,3-dicyano-t-butyl group, a 1,2,3-tricyanopropyl group, a nitromethyl group, a 1-nitroethyl group, a 2-nitroethyl group, a 2-nitroisobutyl group, a 1,2-dinitroethyl group, a 1,3-dinitroisopropyl group, a 2,3-dinitro-t-butyl group, a 1,2,3-trinitropropyl group, and the like, but are not limited thereto.

The alkenyl group may be straight or branched, and the number of carbon atoms thereof is not particularly limited, but is preferably 2 to 20. Examples thereof include a vinyl group, an allyl group, a 1-butenyl group, a 2-butenyl group, a 3-butenyl group, a 1,3-butanedienyl group, a 1-methylvinyl group, a styryl group, a 2,2-diphenylvinyl group, a 1,2-diphenylvinyl group, a 1-methylallyl group, a 1,1-dimethylallyl group, a 2-methylallyl group, a 1-phenylallyl group, a 2-phenylallyl group, a 3-phenylallyl group, a 3,3-diphenylallyl group, a 1,2-dimethylallyl group, a 1-phenyl-1-butenyl group, a 3-phenyl-1-butenyl group, and the like, but are not limited thereto.

The cycloalkyl group is preferably a group having 3 to 20 carbon atoms, which does not cause a steric hindrance. Specific examples thereof include a cyclopentyl group, a cyclohexyl group and the like, but are not limited thereto.

The cycloalkenyl group preferably has 3 to 20 carbon atoms, and more specifically, examples thereof include a ring compound having ethenylene in a pentagonal or hexagonal ring, and the like, but are not limited thereto.

The alkoxy group preferably has 1 to 30 carbon atoms, and more specifically, examples thereof include methoxy, ethoxy, isopropyloxy, and the like, but are not limited thereto.

The aryloxy group preferably has 6 to 60 carbon atoms, and more specifically, examples thereof include phenyloxy, cyclohexyloxy, naphthyloxy, diphenyloxy, and the like, but are not limited thereto.

The alkylamine group preferably has 1 to 30 carbon atoms, and more specifically, examples thereof include a methylamine group, a dimethylamine group, an ethylamine group, a diethylamine group, and the like, but are not limited thereto.

The arylamine group preferably has 5 to 60 carbon atoms, and more specifically, examples thereof include a phenylamine group, a naphthylamine group, a biphenylamine group, an anthracenylamine group, a 3-methyl-phenylamine group, a 4-methyl-naphthylamine group, a 2-methyl-biphenylamine group, a 9-methyl-anthracenylamine group, a diphenylamine group, a phenylnaphthylamine group, a ditolylamine group, a phenyltolylamine group, a triphenylamine group, and the like, but are not limited thereto.

The aryl group may be monocyclic or polycyclic, and the number of carbon atoms thereof is not particularly limited, but is preferably 6 to 60. Examples thereof include a phenyl group, stilbene, a 1-naphthyl group, a 2-naphthyl group, a 1-anthryl group, a 2-anthryl group, a 9-anthryl group, a 1-phenanthryl group, a 2-phenanthryl group, a 3-phenanthryl group, a 4-phenanthryl group, a 9-phenanthryl group, a 1-naphthacenyl group, a 2-naphthacenyl group, a 9-naphthacenyl group, a 1-pyrenyl group, a 2-pyrenyl group, a 4-pyrenyl group, a 2-biphenylyl group, a 3-biphenylyl group, a 4-biphenylyl group, a p-terphenyl-4-yl group, a p-terphenyl-3-yl group, a p-terphenyl-2-yl group, an m-terphenyl-4-yl group, an m-terphenyl-3-yl group, an m-terphenyl-2-yl group, an o-tollyl group, an_ m-tollyl group, a p-tolyl group, a p-t-butylphenyl group, a p-(2-phenylpropyl)phenyl group, a 3-methyl-2-naphthyl group, a 4-methyl-1-naphthyl group, a 4-methyl-1-anthryl group, a 4'-methylbiphenylyl group, a 4"-t-butyl-p-terphenyl-4-yl group, and the like, but are not limited thereto.

The heteroaryl group is a ring group including at least one of O, N, S, Se, and P as a heterogeneous atom, and the number of carbon atoms thereof is not particularly limited, but is preferably 3 to 60. Examples thereof include a 1-pyrolyl group, a 2-pyrolyl group, a 3-pyrolyl group, a pyrazinyl group, a 2-pyridinyl group, a 3-pyridinyl group, a 4-pyridinyl group, a 1-indolyl group, a 2-indolyl group, a 3-indolyl group, a 4-indolyl group, a 5-indolyl group, a 6-indolyl group, a 7-indolyl group, a 1-isoindolyl group, a 2-isoindolyl group, a 3-isoindolyl group, a 4-isoindolyl group, a 5-isoindolyl group, a 6-isoindolyl group, a 7-isoindolyl group, a 2-furyl group, a 3-furyl group, a 2-benzofuranyl group, a 3-benzofuranyl group, a 4-benzofuranyl group, a 5-benzofuranyl group, a 6-benzofuranyl group, a 7-benzofuranyl group, a 1-isobenzofuranyl group, a 3-isobenzofuranyl group, a 4-isobenzofuranyl group, a 5-isobenzofuranyl group, a 6-isobenzofuranyl group, a 7-isobenzofuranyl group, a 2-quinolyl group, a 3-quinolyl group, a 4-quinolyl group, a 5-quinolyl group, a 6-quinolyl group, a 7-quinolyl group, an 8-quinolyl group, a 1-isoquinolyl group, a 3-isoquinolyl group, a 4-isoquinolyl group, a 5-isoquinolyl group, a 6-isoquinolyl group, a 7-isoquinolyl group, an 8-isoquinolyl group, a 2-quinoxalinyl group, a 5-quinoxalinyl group, a 6-quinoxalinyl group, a 1-carbazolyl group, a 2-carbazolyl group, a 3-carbazolyl group, a 4-carbazolyl group, a 9-carbazolyl group, a 1-phenanthridinyl group, a 2-phenanthridinyl group, a 3-phenanthridinyl group, a 4-phenanthridinyl group, a 6-phenanthridinyl group, a 7-phenanthridinyl group, an 8-phenanthridinyl group, a 9-phenanthridinyl group, a 10-phenanthridinyl group, a 1-acridinyl group, a 2-acridinyl group, a 3-acridinyl group, a 4-acridinyl group, a 9-acridinyl group, a 1,7-phenanthrolin-2-yl group, a 1,7-phenanthrolin-3-yl group, a 1,7-phenanthrolin-4-yl group, a 1,7-phenanthrolin-5-yl group, a 1,7-phenanthrolin-6-yl group, a 1,7-phenanthrolin-8-yl group, a 1,7-phenanthrolin-9-yl group, a 1,7-phenanthrolin-10-yl group, a 1,8-phenanthrolin-2-yl group, a 1,8-phenanthrolin-3-yl group, a 1,8-phenanthrolin-4-yl group, a 1,8-phenanthrolin-5-yl group, a 1,8-phenanthrolin-6-yl group, a 1,8-phenanthrolin-7-yl group, a 1,8-phenanthrolin-9-yl group, a 1,8-phenanthrolin-10-yl group, a 1,9-phenanthrolin-2-yl group, a 1,9-phenanthrolin-3-yl group, a 1,9-phenanthrolin-4-yl group, a 1,9-phenanthrolin-5-yl group, a 1,9-phenanthrolin-6-yl group, a 1,9-phenanthrolin-7-yl group, a 1,9-phenanthrolin-8-yl group, a 1,9-phenanthrolin-10-yl group, a 1,10-phenanthrolin-2-yl group, a 1,10-phenanthrolin-3-yl group, a 1,10-phenanthrolin-4-yl group, a 1,10-phenanthrolin-5-yl group, a 2,9-phenanthrolin-1-yl group, a 2,9-phenanthrolin-3-yl group, a 2,9-phenanthrolin-4-yl group, a 2,9-phenanthrolin-5-yl group, a 2,9-phenanthrolin-6-yl group, a 2,9-phenanthrolin-7-yl group, a 2,9-phenanthrolin-8-yl group, a 2,9-phenanthrolin-10-yl group, a 2,8-phenanthrolin-1-yl group, a 2,8-phenanthrolin-3-yl group, a 2,8-phenanthrolin-4-yl group, a 2,8-phenanthrolin-5-yl group, a 2,8-phenanthrolin-6-yl group, a 2,8-phenanthrolin-7-yl group, a 2,8-phenanthrolin-9-yl group, a 2,8-phenanthrolin-10-yl group, a 2,7-phenanthrolin-1-yl group, a 2,7-phenanthrolin-3-yl group, a 2,7-phenanthrolin-4-yl group, a 2,7-phenanthrolin-5-yl group, a 2,7-phenanthrolin-6-yl group, a 2,7-phenanthrolin-8-yl group, a 2,7-phenanthrolin-9-yl group, a 2,7-phenanthrolin-10-yl group, a 1-phenazinyl group, a 2-phenazinyl group, a 1-phenothiazinyl group, a 2-phenothiazinyl group, a 3-phenothiazinyl group, a 4-phenothiazinyl group, a 10-phenothiazinyl group, a 1-phenoxazinyl group, a 2-phenoxazinyl group, a 3-phenoxazinyl group, a 4-phenoxazinyl group, a 10-phenoxazinyl group, a 2-oxazolyl group, a 4-oxazolyl group, a 5-oxazolyl group, a 2-oxadiazolyl group, a 5-oxadiazolyl group, a 3-furazanyl group, a 2-thienyl group, a 3-thienyl group, a 2-methylpyrrol-1-yl group, a 2-methylpyrrol-3-yl group, a 2-methylpyrrol-4-yl group, a 2-methylpyrrol-5-yl group, a 3-methylpyrrol-1-yl group, a 3-methylpyrrol-2-yl group, a 3-methylpyrrol-4-yl group, a 3-methylpyrrol-5-yl group, a 2-t-butylpyrrol-4-yl group, a 3-(2-phenylpropyl)pyrrol-1-yl group, a 2-methyl-1-indolyl group, a 4-methyl-1-indolyl group, a 2-methyl-3-indolyl group, a 4-methyl-3-indolyl group, a 2-t-butyl-1-indolyl group, a 4-t-butyl-1-indolyl group, a 2-t-butyl-3-indolyl group, a 4-t-butyl-3-indolyl group, and the like, but are not limited thereto.

Further, the heteroaryl group are preferably compounds having the following structural formulae, but are not limited thereto.

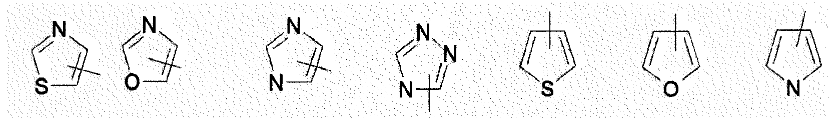

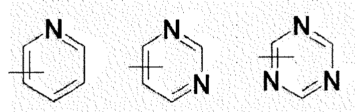

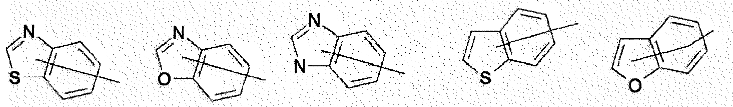

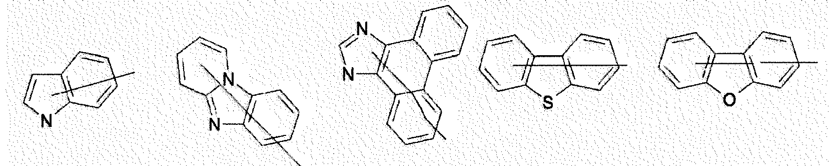

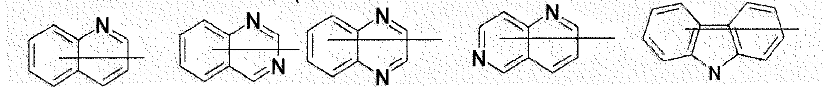

Examples of the halogen group include fluorine, chlorine, bromine, iodine, and the like, but are not limited thereto.

In an exemplary embodiment of the present specification, an organic material layer including the above-described heat-treated, light-treated, or acid-treated compound is an anode buffer layer. Specifically, the anode buffer layer may serve as a hole transport layer.

In an exemplary embodiment of the present specification, the organic material layer including the heat-treated, light-treated, or acid-treated compound may be provided between a photoactive layer and an anode. In an exemplary embodiment of the present specification, the organic material layer including the heat-treated, light-treated, or acid-treated compound is a photoactive layer.

In an exemplary embodiment, the photoactive layer includes an electron donor material and an electron acceptor material, and the electron donor material includes the heat-treated, light-treated, or acid-treated compound.

In another exemplary embodiment, the photoactive layer includes an organic material layer having three or more layers, the organic material layer includes: a p-type organic material layer; an n-p-type organic material layer; and an n-type organic material layer, and

one or more layers of the p-type organic material layer and the n-p-type organic material layer include a heat-treated, light-treated, or acid-treated compound. The thickness of the organic photoactive layer may be 10 to 10,000 Å, but is not limited thereto.

In the present specification, charge extraction and charge transporting may be increased by forming the photoactive layer as a three layer structure including: a p-type organic material layer; an n-p-type organic material layer; and an n-type organic material layer, to produce a new hole transport layer and a new electron transport layer, and accordingly, the total charge mobility may be increased. Furthermore, the energy level may be freely adjusted, and accordingly, holes and electrons may be easily transferred, and an ideal p-n structure may be formed.

In the photoactive layer having a three layer structure, the thickness of the n-type organic material layer may be 20 to 400 nm, the thickness of the n-p-type organic material layer may be 20 to 400 nm, and the thickness of the p-type organic material layer may be more than 0 and 300 nm or less, but the thicknesses thereof are not limited thereto.

In the present specification, the photoactive layer may have a structure sequentially including: a p-type organic material layer; an n-p-type organic material layer; and an n-type organic material layer. In this case, the electron donor material may be a compound having the group capable of forming a hydrogen bond as described above, and more specifically, may include the compound represented by Formula 1.

In the present specification, the electron acceptor material may be fullerene, a fullerene derivative, vasocuproin, a semiconductor element, a semiconductor compound, or a combination thereof, and specifically, may be phenyl C61-butyric acid methyl ester (PC₆₁BM), or phenyl C71-butyric acid methyl ester (PC₇₁BM), but is not limited thereto.

In the n-p-type organic material layer in the present specification, the electron donor material and the electron acceptor material may be mixed at a ratio (w/w) of 1:10 to 10:1, and may be subjected to heat treatment at 30 to 400°C for 1 second to 48 hours or to surface treatment with an acid such as acetic acid, HCl and H₂SO₄ in order to maximize characteristics after the materials are mixed, but the treatment is not limited thereto.

An organic solar cell according to an exemplary embodiment of the present specification includes an anode, a photoactive layer, and a cathode electrode. The organic solar cell may further include a substrate, a hole transport layer, and/or an electron transport layer.

In an exemplary embodiment of the present specification, the organic solar cell has a normal structure. The normal structure may mean that an anode is formed on a substrate.

In an exemplary embodiment of the present specification, the organic solar cell has an inverted structure. When the organic solar cell according to an exemplary embodiment of the present specification has an inverted structure, the inverted structure may mean that a cathode is formed on a substrate, and the anode may be silver (Ag) or MoO₃/Al.

The organic solar cell according to the present specification may be prepared by a material and a method known in the art, except that an organic material layer as well as a photoactive layer includes the above-described compound of the present specification, that is, the compound having a group capable of forming a hydrogen bond, for example, the compound represented by Formula 2.

A method for preparing an organic solar cell according to the present invention includes: forming an anode or a cathode on a substrate; forming an organic material layer having one or more layers on the anode or the cathode; and forming a cathode or an anode on the organic material layer having one or more layers, in which the forming of the organic material layer having one or more layers includes: forming a thin film by using a compound including a leaving group which is removed by heat treatment, light treatment, or acid treatment; and subjecting the formed thin film to heat treatment, light treatment, or acid treatment, and the heat-treated, light-treated, or acid-treated thin film includes a compound represented by Formula 2-1 or Formula 2-2.

In inducing the compound including a leaving group to form a hydrogen bond again by heat treatment, light treatment, or acid treatment in the present specification, the heat treatment temperature or time is not particularly limited, and may be selected according to the kinds of substituents.

In an exemplary embodiment of the present specification, the heat treatment may be performed at a temperature of 20 to 400°C for 1 second to 48 hours. In another exemplary embodiment, the temperature is preferably in a range of 20 to 350°C, and it is preferred that the treatment is performed within a time of 1 minute to 2 hours. Specifically, it is preferred that the treatment is performed at a temperature of 150 to 300°C, more preferably 180 to 280°C, and that the treatment is performed within 1 hour.

In performing light treatment, the exposure time is not particularly limited, but is generally preferably less than 30 minutes.

The acid treatment may be performed by a method of treating the surface with an acid such as acetic acid, HCl, and H₂SO₄, but is not limited thereto.

The substrate may be a glass substrate or a transparent plastic substrate having excellent transparency, surface smoothness, easiness in handling, and water proof property, but is not limited thereto, and is not limited as long as the substrate is a substrate typically used in the organic solar cell. Specific examples thereof include glass, polyethylene terephthalate (PET), polyethylene naphthalate (PEN), polypropylene (PP), polyimide (PI), triacetyl cellulose (TAC), and the like, but are not limited thereto.

The anode may include a material which is transparent and excellent in conductivity, but is not limited thereto. Specifically, the material thereof may be indium tin oxide (ITO), tin oxide (SnO₂), and zinc oxide (ZnO), but is not limited thereto.

The cathode may include a metal having a small work function, but is not limited thereto. Specifically, the material thereof may be a metal such as lithium, magnesium, and aluminum or an alloy thereof, and a material having a multi-layer structure such as Al;Li, Al:BaF₂, and Al:BaF₂:Ba, but is not limited thereto.

The hole transport layer and/or the electron transport layer materials may be a material efficiently transferring electrons and holes to the photoactive layer to increase a possibility of movement of generated charges to the electrode, but are not particularly limited thereto. The hole transport layer material may be PEDOT:PSS (Poly(3,4-ethylenediocythiophene) doped with poly(styrenesulfonic acid)), molybdenum oxide (MoOₓ) ; vanadium oxide (V₂O₅) ; nickel oxide (NiO); and tungsten oxide (WOₓ), and the like, but is not limited thereto.

The electron transport layer material may be electron-extracting metal oxides, and specifically, may be a metal complex of 8-hydroxyquinoline; a complex including Alq₃; a metal complex including Liq; LiF; Ca; titanium oxide (TiOₓ); zinc oxide (ZnO); cesium carbonate (CS₂CO₃), and the like, but is not limited thereto.

In the organic solar cell, the anode electrode, the hole transport layer, the photoactive layer, the electron transport layer, and the cathode electrode may be disposed in this order, or the cathode electrode, the electron transport layer, the photoactive layer, the hole transport layer, and the anode electrode may be disposed in this order, but the order is not limited thereto.

In another exemplary embodiment of the present specification, the organic solar cell has a tandem structure. In this case, the organic solar cell may include one layer or two or more layers of the photoactive layer.

Hereinafter, preferred Examples will be provided for better understanding the present specification. However, the following Examples are provided for illustrative purposes only, and the scope of the present specification is not limited thereby.

### <Example>

### <Preparation Example 1> Preparation of Formula 1-1

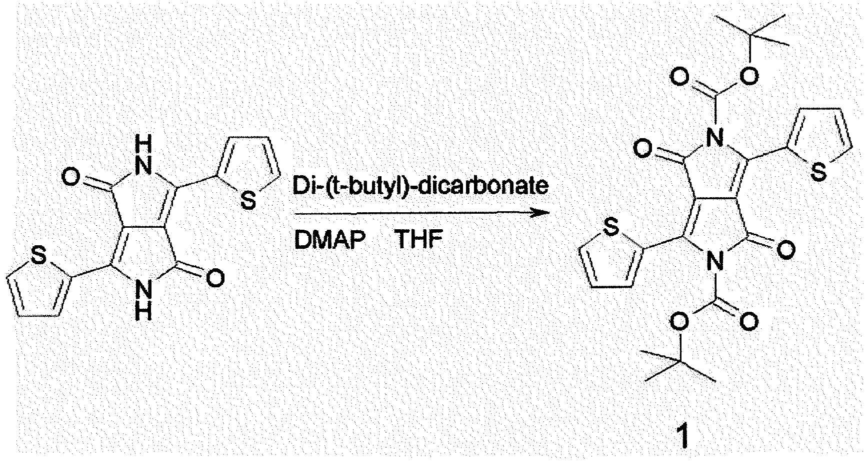

### (1) Synthesis of Compound 1

TPP (4.0 g, 13.3 mmol) was dissolved in an anhydride THF in a 2-neck round bottom flask, and then, dimethylamonopyridine as a catalyst was added thereto, and the resulting mixture was stirred for about 1 hour. And then, di-(t-butyl)-dicarbonate (7.26 g, 33.2 mmol) was dissolved well in the anhydride THF, the solution was added to the flask under a nitrogen condition by using a syringe, and the resulting mixture was stirred at R.T. for 12 hours. The reaction was confirmed by TLC, and after the reaction was terminated, the THF was placed under reduced pressure. Silica packing was achieved by using hexane + Et3N as an eluent, and the reaction mixture was loaded, and then flowed at Hx : EA = 4 : 1 to remove impurities, and chloroform was flowed. After the column chromatography, pressure was reduced to remove the solvent, and the residue was washed with hexane and dried well in a vacuum oven, thereby obtaining 4.0 g of a dark brown solid compound t-Boc TPP (yield: 60%).

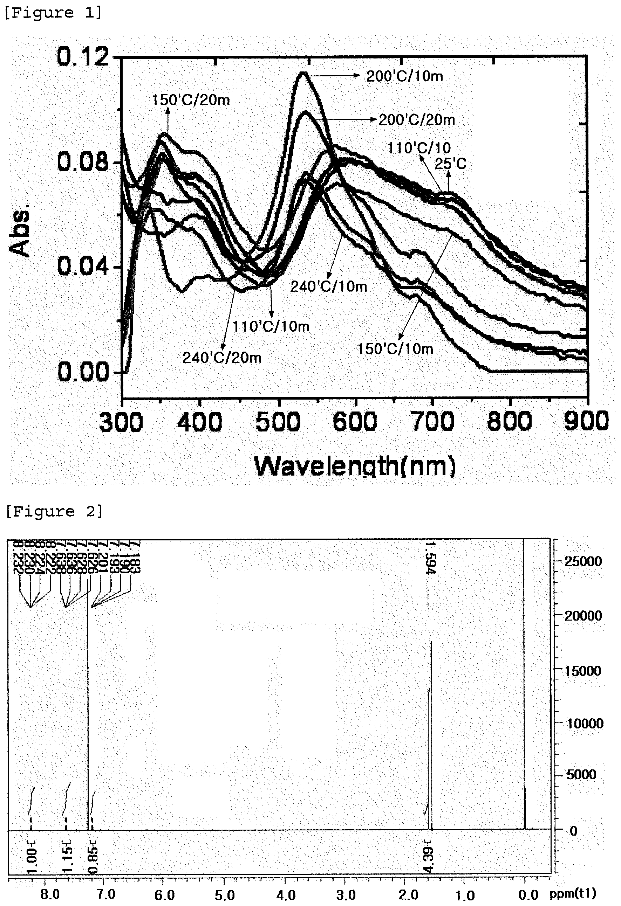

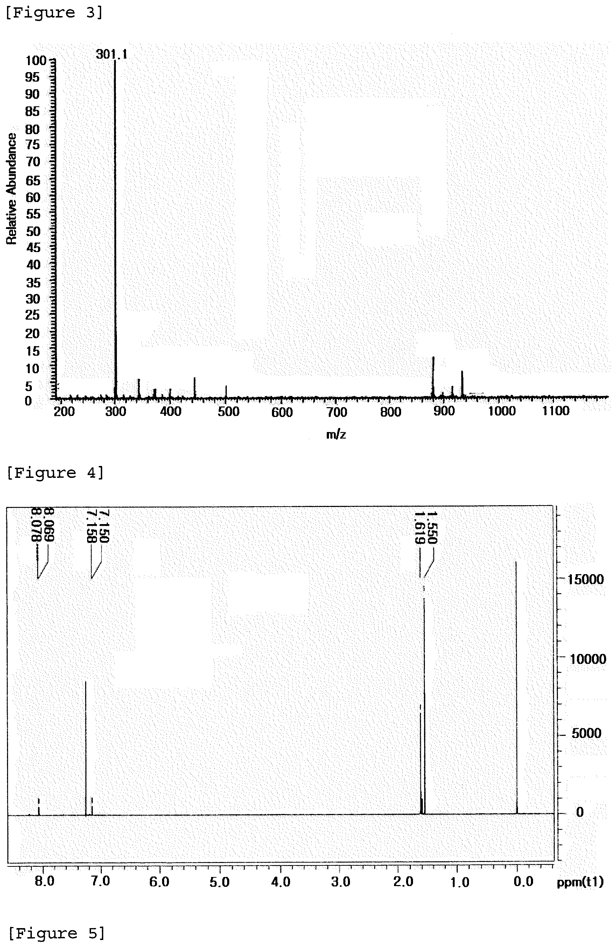

The NMR result of Compound 1 is illustrated in the following FIG. 2, and the MS data thereof are illustrated in the following FIG. 3.

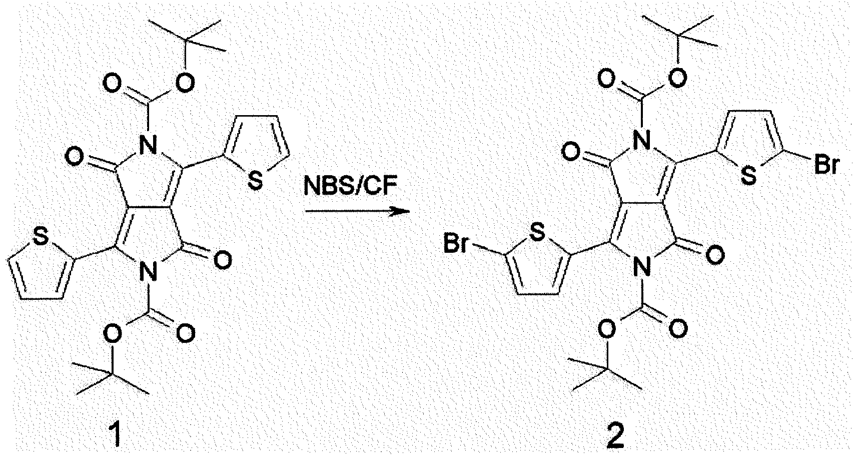

### (2) Synthesis of Compound 2

The t-Boc TPP (2.36 g, 4.71 mmol) was added to CF in a 2-neck round bottom flask, and the mixture was stirred at room temperature. Light protection was performed by using an aluminum foil, and then NBS (2.09 g, 2.5 eq.) was added thereto and the mixture was stirred for 40 hours or more. It was confirmed through TLC whether the reaction proceeded, and if the reaction proceeded, the crude mixture was immediately evaporated and recrystallization was performed by using CF/EtOH, thereby obtaining 1.6 g (yield: 50%) of Compound 2 having a dark brown needle shape.

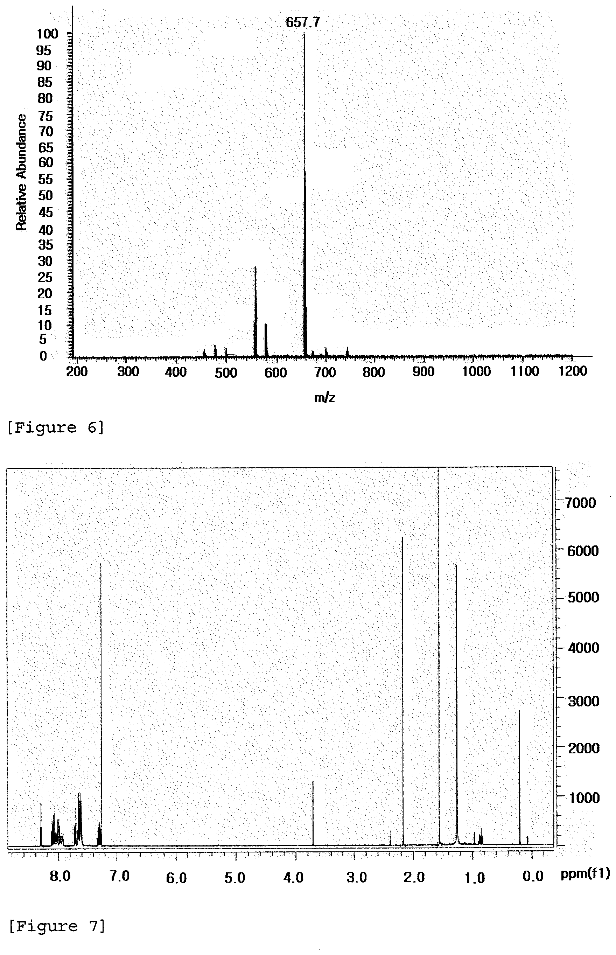

The NMR result of Compound 2 is illustrated in the following FIG. 4, and the MS data thereof are illustrated in the following FIG. 5.

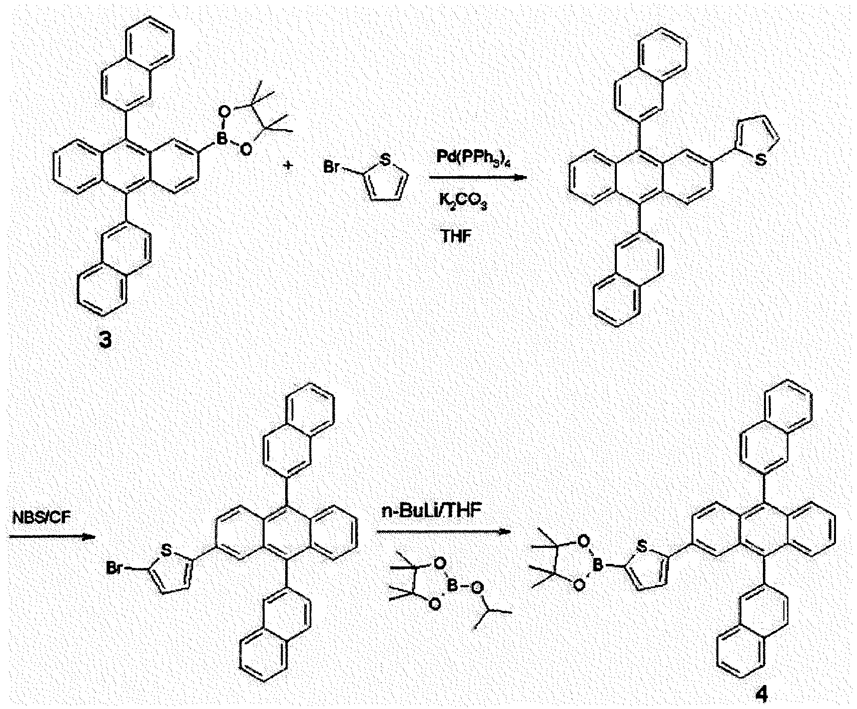

### (3) Synthesis of Compound 4

Compound 3 (5 g, 8.9844 mmol), 2-bromothiophene (2.19 g, 13.4766 mmol), Pd(PPh₃)₄ (0.46, 3 mol%), and 2M K₂CO₃ (10 ml) were put into a 2-neck round bottom flask, THF was added thereto, and the resulting mixture was stirred while the temperature was increased. After the reaction proceeded, an organic layer extracted with MC and water was dried over MgSO₄, and then a powder was obtained by using a silica column (eluent: Hx/MC = 10/3) (yield: 78%).

The well dried powder (3.0 g, 5.8516 mmol) was added to CF, and the resulting mixture was stirred at room temperature. Light protection was performed by using an aluminum foil, and then NBS (1.14 g, 1.05 eq.) was added thereto and the mixture was stirred for 18 hours. After confirmation with TLC, a powder was obtained by using a silica column (eluent: Hx/MC = 10/3) (yield: 87%).

The well dried powder (3.14 g, 5.3000 mmol) was dissolved in 250 ml of tetrahydrofuran, the temperature was lowered to -70°C, n-BuLi (2.5 M in hexane, 3.2 ml) was added thereto, and the resulting mixture was stirred for 1 hour. 2-Isopropoxy-4,4,5,5-tetramethyl-1,3,2-dioxaborolane (1.48 g) was added thereto. After the reaction, a powder was obtained by using a silica column (eluent: Hx/MC = 10/5) (yield: 67%).

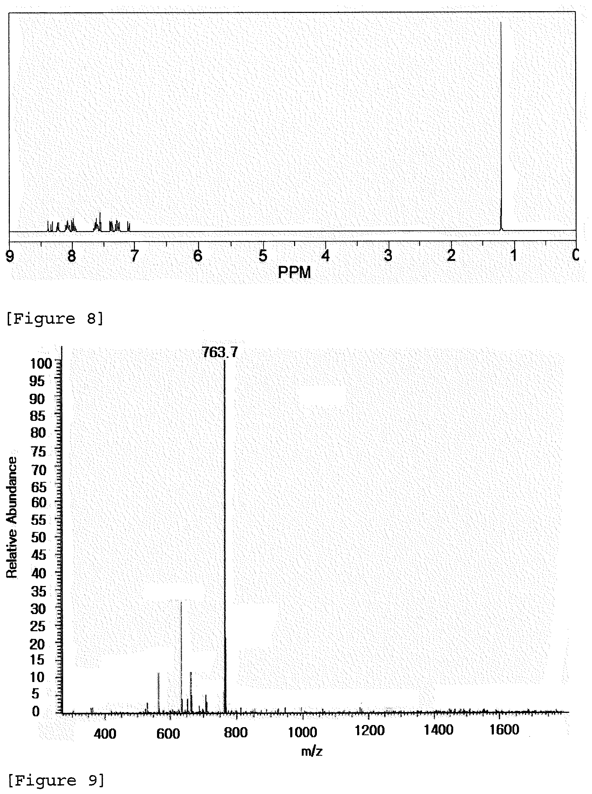

The NMR result of Compound 3 is illustrated in the following FIGS. 6 and 7.

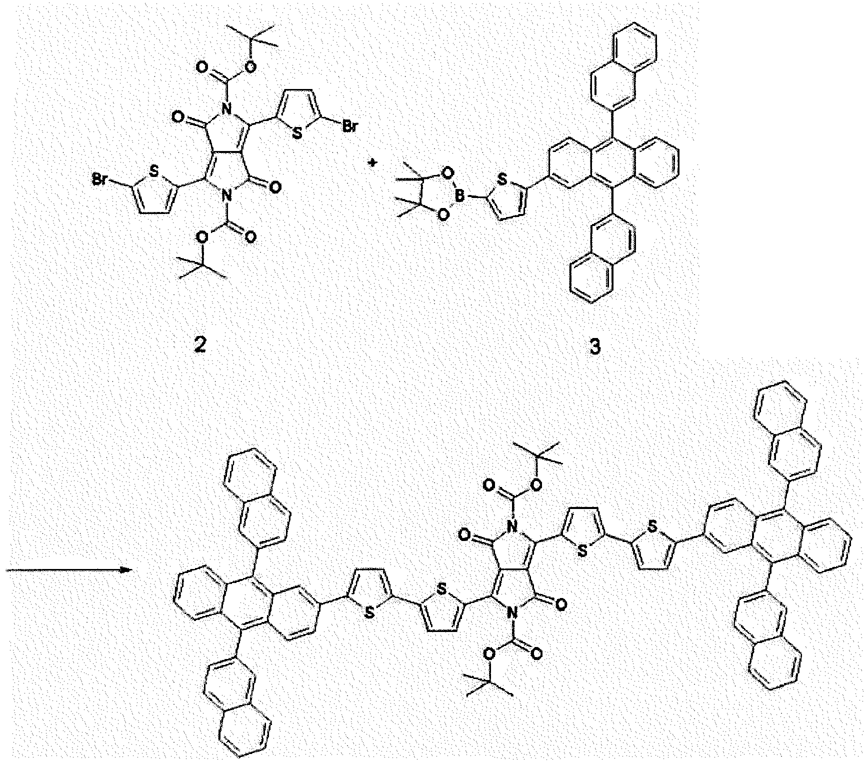

### (4) Synthesis of Formula 1-1

Compound 2 (0.5 g, 0.7594 mmol) and Compound 3 (1.12 g, 1.7467 mmol) were added to 200 ml of toluene in a 250 ml-2-neck round bottom flask, and the resulting mixture was stirred. The temperature was increased up to 50°C, and then Pd(0) (0.26 mg, 3 mol%) was added thereto. The mixture was stirred for several minutes, 2 M aq. K₂CO₃ was added thereto, and the resulting mixture was stirred while the temperature was increased to 90°C. After the reaction, the temperature was lowered to room temperature, pressure was reduced at 80°C to evaporate the solvent, the residue was washed several times with water, and then the target compound was obtained by crystallization with MC/EtOH (yield: 83%).

### <Preparation Example 2> Preparation of Formula 1-2

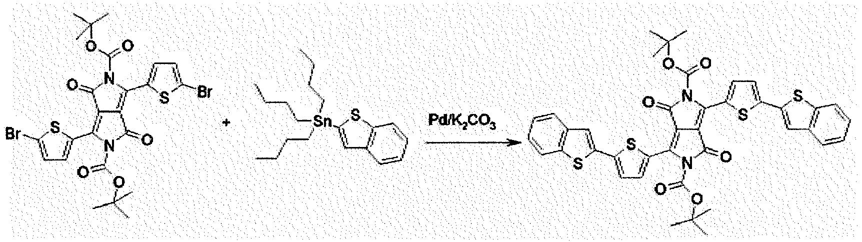

Compound 2 (0.62 g, 0.94 mmol), 2-tributylstannylbenzo[b]thiophene (1.0 g, 2.39 mmol), and Pd(PPh₃)₄ (3 mol% based on the Br compound) was added to 100 ml of toluene in a 2-neck round bottom flask, and the resulting mixture was stirred at 100°C for 3 hours. The precipitate was filtered and the target compound was obtained through short column and recrystallization (yield: 48%) .

The MS data of Formula 1-2 are illustrated in the following FIG. 8.

### <Experimental Example>

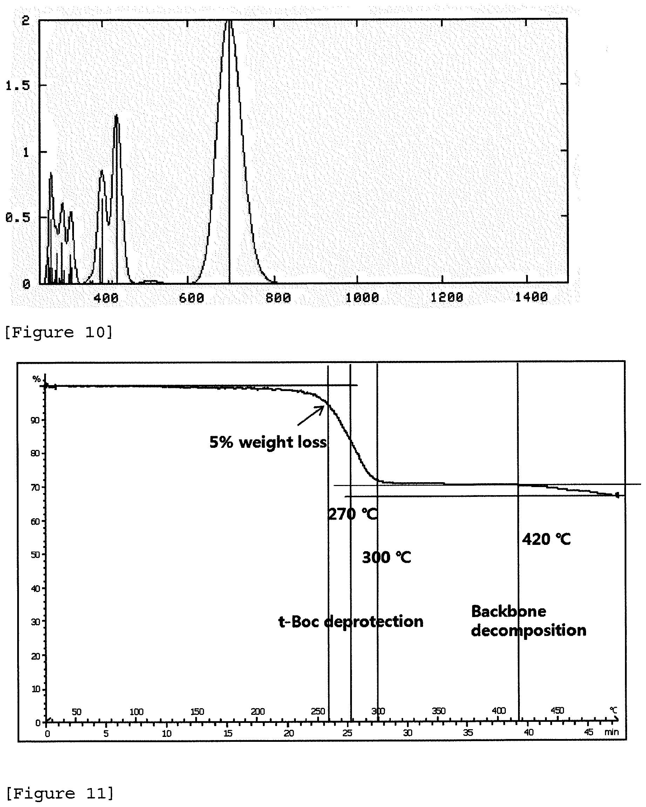

The UV-spectrum of Formula 1-1 is illustrated in the following FIG. 9.

The characteristics of Formula 1-1 are as follows.
- Band gap: 1.79 eV
- Electrochemical characteristics: HOMO(-5.33 eV), LUMO (-3.55 eV)
- Torsion angle of a, b, c, and d: a (20.886 degrees), b (10.65 degrees), c (14.5 degrees), and d (86.5 degrees) 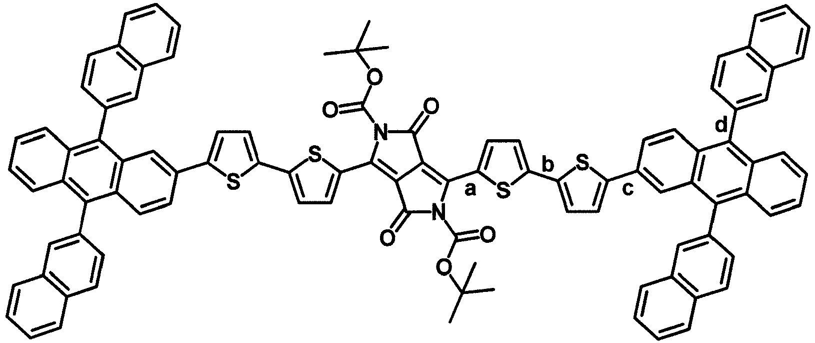
- Planarity: 1.6188

From the electrochemical characteristics and band gap of the compound, it can be confirmed that the compound may be applied to an organic solar cell.

FIG. 1 a view illustrating UV spectrum results of Formula 1-1 after a heat treatment process according to an exemplary embodiment of the present invention.

From the results of FIG. 1, the heat treatment at a predetermined temperature or more may induce the blue-shift of a material. This phenomenon results from the fact that the p-p stacking is suppressed by removing the side chain, but as a strong molecular interaction (hydrogen bond) occurs, the band gap is increased due to the intermolecular twisted phenomenon. However, after the heat treatment process, a structure such a polymer having good crystallinity may be formed and used as a new hole transport layer and a new electron donor layer to enhance charge mobility and stability, thereby enhancing the efficiency of an organic solar cell.

FIG. 10 is a view illustrating a thermogravimetry analysis of Formula 1-1.

From the result of FIG. 10, it can be confirmed that a t-butoxycarbonyl group of Formula 1-1 has been removed at a heat treatment temperature of 260 to 300°C. However, when the heat treatment temperature exceeds 420°C, the core structure may be decomposed.

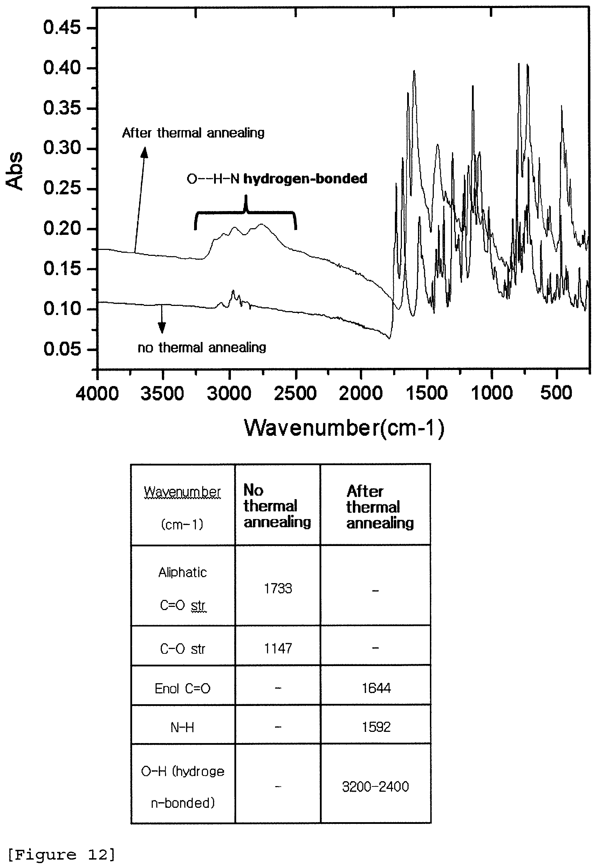

FIG. 11 is a view illustrating an IR spectrum of Formula 1-1.

From the result of FIG. 11, it can be confirmed that after the heat treatment, hydrogen is substituted while a leaving group is removed from Formula 1-1, and the intensity of the hydrogen bond, which the hydrogen and a group capable of forming a hydrogen bond of the compound of Formula 1-1 form, becomes strong.

### Comparative Example 1.

Formula 1-1 and PCBM were dissolved in a ratio of 1:2 in chlorobenzene (CB) to prepare a composite solution. In this case, the concentration was adjusted to 2.0 wt%, and an organic solar cell was made to have a structure of ITO/PEDOT:PSS/a photoactive layer/Al. The glass substrate coated with the ITO was ultrasonically cleaned by using distilled water, acetone, and 2-propanol, the ITO surface was treated with ozone for 10 minutes, spin coating was performed by using PEDOT:PSS (baytrom P) in a thickness of 45 nm, and heat treatment was performed at 120°C for 10 minutes. For coating the photoactive layer, the compound-PCBM composite solution was filtered with a 0.45 µm-PP syringe filter, and then an organic solar cell was prepared by spin-coating the solution to deposit Al in a thickness of 200 nm.

### Experimental Example 1.

The compound was spin-coated, and then an organic solar cell was prepared by the same method, except that heat treatment was performed by using a thermal evaporator under a vacuum of 3 x 10⁻⁸ torr.

**[Table 1]**

| | V_{oc} (V) | J_{sc} (mA/cm²) | FF (%) | PCE (%) |
|---|---|---|---|---|
| Comparative Example 1 | 0.654 | 6.606 | 0.582 | 2.52 |
| Experimental Example 1 | 0.680 | 8.821 | 0.584 | 3.5 |

In Table 1, V_{oc} means an open-circuit voltage, J_{sc} means a short-circuit current, FF means a fill factor, and PCE means an energy conversion efficiency. The open-circuit voltage and the short-circuit current are the intercept of the X-axis and the Y-axis in the fourth quadrant of the voltage-current density curve, respectively, and when the two values becomes high, the efficiency of the solar cell is desirably increased. Furthermore, the fill factor is a value obtained by dividing the area of a rectangle, which may be drawn inside the curve, by the product of the short-circuit current and the open-circuit voltage. When the three values are divided by the intensity of light irradiated, the energy conversion efficiency may be obtained, and the higher value is preferred.

FIG. 12 is a view illustrating a current density according to the voltage of an organic solar cell according to Comparative Example 1.

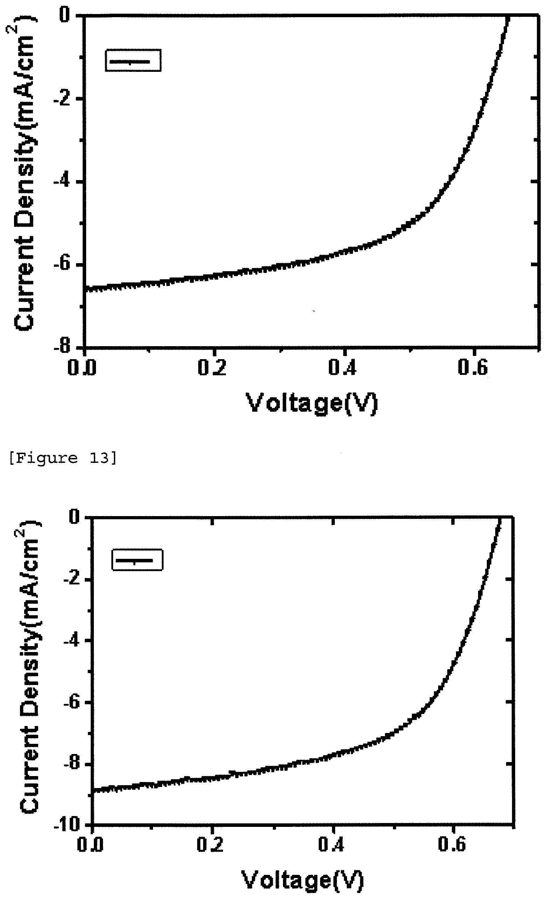

FIG. 13 is a view illustrating a current density according to the voltage of an organic solar cell according to Experimental Example 1.

From the results of Experimental Example 1 and Comparative Example 1, it can be confirmed that hydrogen is substituted while the leaving group is removed by heat treatment, light treatment, or acid treatment, and the hydrogen and a group capable of forming a hydrogen bond combine with each other to form a hydrogen bond, and an organic solar cell with high efficiency may be provided due to high charge mobility through the hydrogen bond.

## Claims

1. An organic solar cell comprising an anode, a cathode, and an organic material layer having one or more layers disposed between the anode and the cathode, **characterized in that** at least one of the one or more layers of the organic material layer comprises a compound represented by the following Formula 2-1 or Formula 2-2:

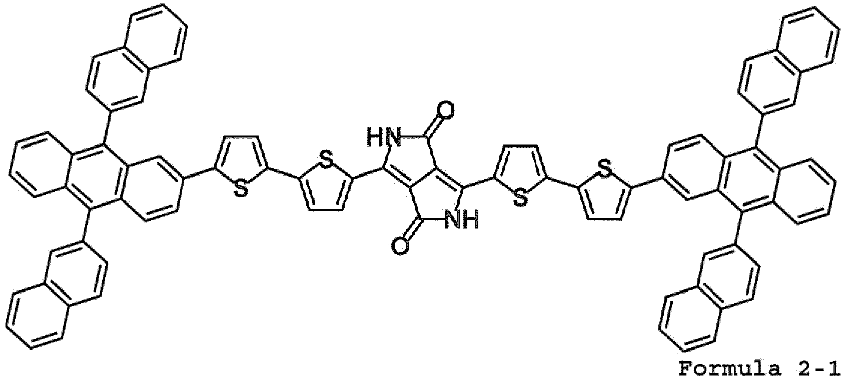

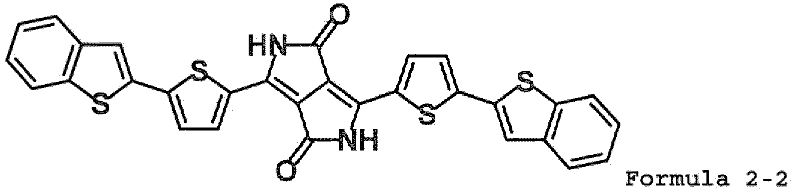

2. An organic solar cell according to Claim 1, wherein the organic material layer comprising the compound represented by the Formula 2-1 or Formula 2-2 is an anode buffer layer.

3. An organic solar cell according to Claim 1, wherein the organic material layer comprising the compound represented by the Formula 2-1 or Formula 2-2 is a photoactive layer.

4. An organic solar cell according to Claim 3, wherein the photoactive layer comprises an electron donor material and an electron acceptor material, and
the electron donor material comprises the compound represented by the Formula 2-1 or Formula 2-2.

5. An organic solar cell according to Claim 3, wherein the photoactive layer comprises an organic material layer having three or more layers,
the organic material layer comprises an n-type organic material layer, an n-p-type organic material layer, and a p-type organic material layer; and
one or more layers of the p-type organic material layer and the n-p-type organic material layer comprises the compound represented by the Formula 2-1 or Formula 2-2.

6. An organic solar cell according to Claim 1, wherein the organic solar cell further comprises one or more of a hole transport layer and an electron transport layer.

7. A method for preparing an organic solar cell comprising the steps of:
forming an anode or a cathode on a substrate;
forming an organic material layer having one or more layers on the anode or the cathode; and
forming a cathode or an anode on the organic material layer,
wherein the forming of the organic material layer having one or more layers comprises:
forming a thin film by using a compound including a leaving group to be removed by heat treatment, light treatment or acid treatment; and
subjecting the formed thin film to heat treatment, light treatment or acid treatment, and **characterized in that** the heat-treated, light-treated or acid-treated thin film comprises a compound represented by Formula 2-1 or Formula 2-2:

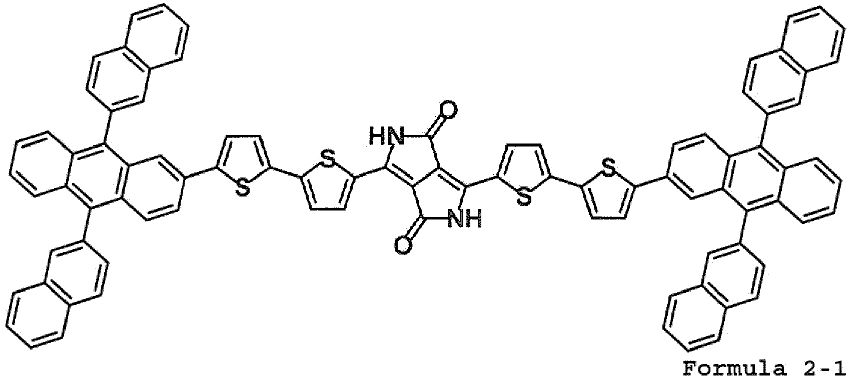

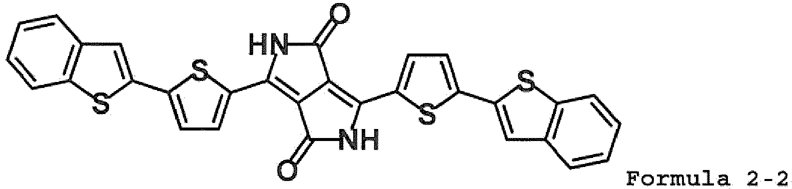

8. A method according to Claim 7, wherein the heat treatment is performed at a temperature of 20 to 400°C for 1 second to 48 hours.

## Patentansprüche

1. Organische Solarzelle, die eine Anode, eine Kathode und eine organische Materialschicht mit ein oder mehr Schichten, die zwischen der Anode und der Kathode angeordnet sind, umfasst,
**dadurch gekennzeichnet, dass** mindestens eine der ein oder mehr Schichten der organischen Materialschicht eine Verbindung mit der folgenden Formel 2-1 oder Formel 2-2 umfasst:

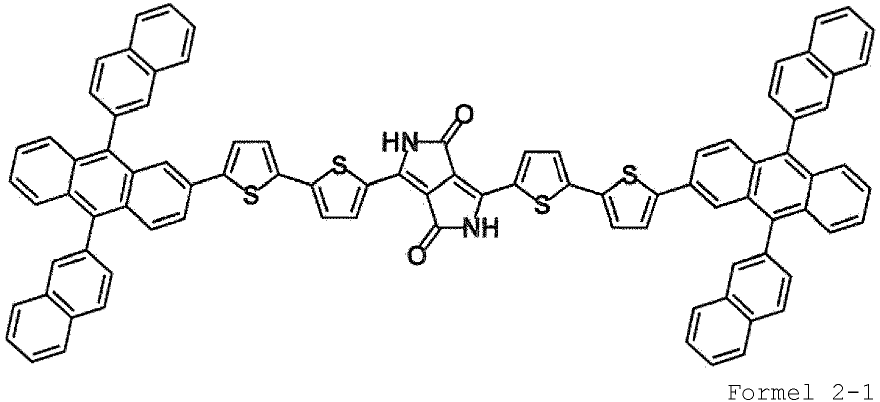

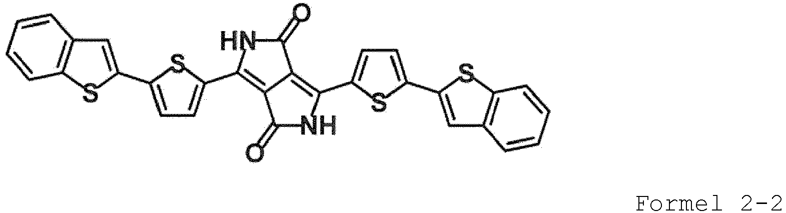

2. Organische Solarzelle gemäß Anspruch 1, wobei die organische Materialschicht, die die Verbindung mit der Formel 2-1 oder Formel 2-2 umfasst, eine Anodenpufferschicht ist.

3. Organische Solarzelle gemäß Anspruch 1, wobei die organische Materialschicht, die die Verbindung mit der Formel 2-1 oder Formel 2-2 umfasst, eine photoaktive Schicht ist.

4. Organische Solarzelle gemäß Anspruch 3, wobei die photoaktive Schicht ein Elektronendonormaterial und ein Elektronenakzeptormaterial umfasst, und
das Elektronendonormaterial die Verbindung mit der Formel 2-1 oder Formel 2-2 umfasst.

5. Organische Solarzelle gemäß Anspruch 3, wobei die photoaktive Schicht eine organische Materialschicht mit drei oder mehr Schichten umfasst,
die organische Materialschicht eine organische Materialschicht vom n-Typ, eine organische Materialschicht vom n-p-Typ und eine organische Materialschicht vom p-Typ umfasst; und
ein oder mehr Schichten der organischen Materialschicht vom p-Typ und der organischen Materialschicht vom n-p-Typ die Verbindung mit der Formel 2-1 oder Formel 2-2 umfassen.

6. Organische Solarzelle gemäß Anspruch 1, wobei die organische Solarzelle ferner ein oder mehr Vertreter aus einer Lochtransportschicht und einer Elektronentransportschicht umfasst.

7. Verfahren zur Herstellung einer organischen Solarzelle, welches die folgenden Schritte umfasst:
eine Anode oder eine Kathode wird auf einem Träger ausgebildet;
eine organische Materialschicht mit ein oder mehr Schichten wird auf der Anode oder der Kathode ausgebildet; und
eine Kathode oder eine Anode wird auf der organischen Materialschicht ausgebildet,
wobei das Ausbilden der organischen Materialschicht mit ein oder mehr Schichten folgendes umfasst:
ein dünner Film wird unter Verwendung eine Verbindung, die eine durch Wärmebehandlung, Lichtbehandlung oder Säurebehandlung zu entfernende Abgangsgruppe einschließt, ausgebildet; und
der ausgebildete dünne Film wird einer Wärmebehandlung, Lichtbehandlung oder Säurebehandlung ausgesetzt, und
**dadurch gekennzeichnet, dass** der wärmebehandelte, lichtbehandelte oder säurebehandelte dünne Film eine Verbindung mit der folgenden Formel 2-1 oder Formel 2-2 umfasst:

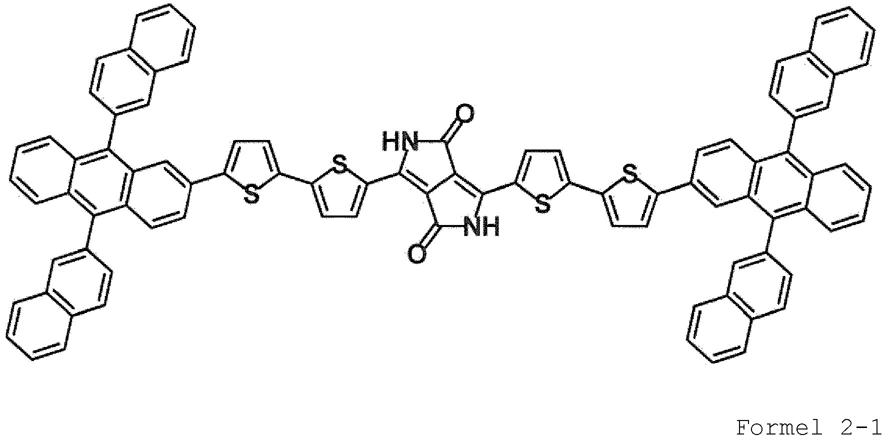

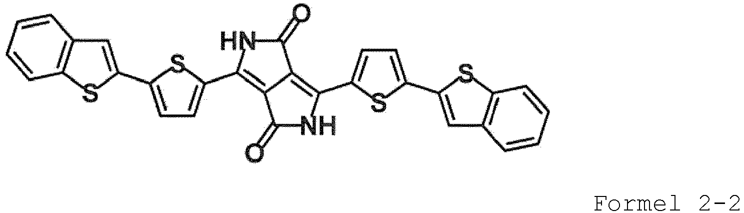

8. Verfahren gemäß Anspruch 7, bei dem die Wärmebehandlung bei einer Temperatur von 20 bis 400°C für 1 Sekunde bis 48 Stunden durchgeführt wird.

## Revendications

1. Cellule solaire organique comprenant une anode, une cathode et une couche de matière organique présentant une ou plusieurs couches disposées entre l'anode et la cathode,
**caractérisée en ce qu'**au moins une des une ou plusieurs couches de la couche de matière organique comprend un composé représenté par la formule 2-1 ou la formule 2-2 suivante :

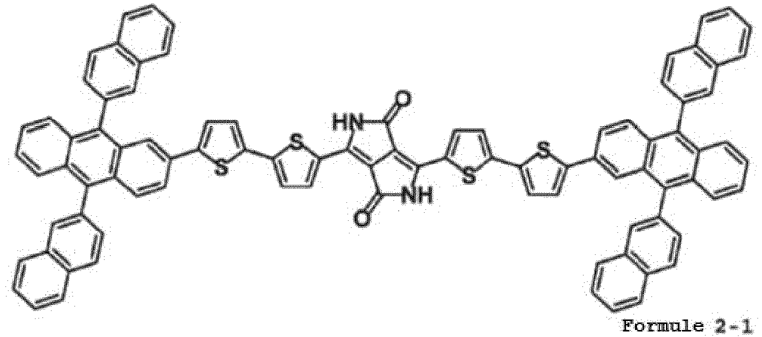

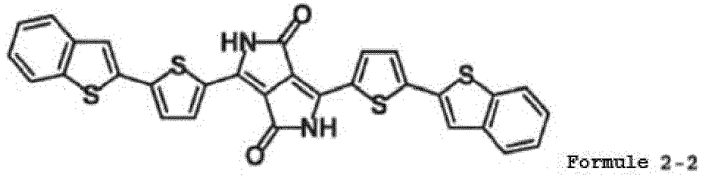

2. Cellule solaire organique selon la revendication 1, dans laquelle la couche de matière organique comprenant le composé représenté par la formule 2-1 ou la formule 2-2 est une couche tampon d'anode.

3. Cellule solaire organique selon la revendication 1, dans laquelle la couche de matière organique comprenant le composé représenté par la formule 2-1 ou la formule 2-2 est une couche photoactive.

4. Cellule solaire organique selon la revendication 3, dans laquelle la couche photoactive comprend une matière donneuse d'électrons et une matière accepteuse d'électrons, et
la matière donneuse d'électrons comprend le composé représenté par la formule 2-1 ou la formule 2-2.

5. Cellule solaire organique selon la revendication 3, dans laquelle la couche photoactive comprend une couche de matière organique présentant trois couches ou plus,
la couche de matière organique comprend une couche de matière organique de type n, une couche de matière organique de type n-p, et une couche de matière organique de type p ; et
une ou plusieurs couches de la couche de matière organique de type p et de la couche de matière organique de type n-p comprennent le composé représenté par la formule 2-1 ou la formule 2-2.

6. Cellule solaire organique selon la revendication 1, dans laquelle la cellule solaire organique comprend en outre une ou plusieurs d'une couche de transport de trous et d'une couche de transport d'électrons.

7. Procédé de préparation d'une cellule solaire organique comprenant les étapes consistant à :
former une anode ou une cathode sur un substrat ;
former une couche de matière organique présentant une ou plusieurs couches sur l'anode ou la cathode ; et
former une cathode ou une anode sur la couche de matière organique,
dans lequel la formation de la couche de matière organique présentant une ou plusieurs couches comprend les étapes consistant à :
former un film mince en utilisant un composé incluant un groupe clivant à éliminer par traitement thermique, traitement à la lumière ou traitement par acide ; et
soumettre le film mince formé à un traitement thermique, un traitement à la lumière ou un traitement par acide, et **caractérisé en ce que**
le film mince traité thermiquement, traité à la lumière ou traité par acide comprend un composé représenté par la formule 2-1 ou la formule 2-2 :

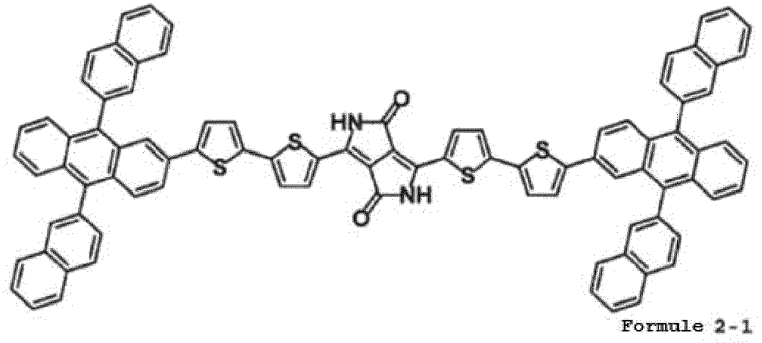

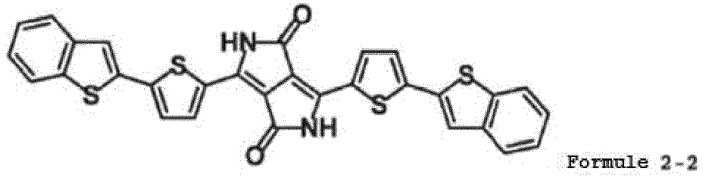

8. Procédé selon la revendication 7, dans lequel le traitement thermique est effectué à une température de 20 à 400 °C pendant 1 seconde à 48 heures.
